# EUROPEAN PATENT SPECIFICATION

(11) **EP 2 501 354 B1**
(45) Date of publication and mention of the grant of the patent: **08.04.2015**
(21) Application number: 09760514.1
(22) Date of filing: 20.11.2009
(51) Int. Cl.: A61J 1/20

(54) **MEDICAL DEVICE CONNECTOR**
STECKVERBINDER FÜR EINE MEDIZINISCHE VORRICHTUNG
RACCORD DE DISPOSITIF MÉDICAL

(43) Date of publication of application: 26.09.2012
(73) Proprietor: Carmel Pharma AB, 402 28 Göteborg (SE)
(72) Inventor: NORD, Lars, S-413 21 Göteborg (SE); CEDERSCHIÖLD, Alexander, S-412 57 Göteborg (SE)
(74) Representative: Valea AB
(86) International application number: PCT/EP2009/065561
(87) International publication number: WO 2011/060828

(56) References cited:
- WO-A1-2006/103074
- WO-A1-2008/136720
- WO-A1-2010/069361
- WO-A2-2007/040396
- DE-B- 1 005 685
- FR-A1- 2 884 723
- US-A- 5 139 483

## Description

### TECHNICAL FIELD

The present invention relates to a connection site for a medical device having a neck element with at least one guiding track. The guiding track has a lock edge for cooperative engagement with a lock protrusion of a second medical device.

### BACKGROUND OF THE INVENTION

Administration of hazardous medicaments such as cytotoxins and the like has long been a nuisance to the personal which on daily basis administrate the hazardous medicaments. During preparation of medicaments, administration or after treatment, nursing personal is exposed to the risk of contamination from the hazardous medicaments. Such contamination may be in the form of liquid, aerosols, or vapours, medicaments, derived from spillage due to ill handling or just wrong handling of equipments or instruments. Leakage from technical equipment which has been used right is however also a problem, even if leakage occur in very small doses. Due to long exposure to hazardous medicaments nursing personal can still be ill from very small quantities of hazardous medicaments. It is therefore important to minimize leakage and minimize the risk of leakage.

One specific hazardous step is when e.g. nursing personal is transferring a medicament from one fluid container to another; such transfer usually involves the use of a piercing member such as a needle. To protect the nursing personal involved, piercing member protection devices are commonly used. Such devices are arranged to protect the user, not only from contamination but also from accidentally piercing themselves or any other third persons. One example of such piercing member protection device, having a needle, is disclosed in U. S. Patent No. 4,564, 054 (Gustavsson).

Piercing devices, such as the ones described in the U. S. Patent No. 4,564, 054 (Gustavsson) generally requires a mating connector or adaptor to enable assembly with a vial to prevent leakage. To enable a firm connection with e.g. piercing devices, medical device connectors, also referred to as medical device adaptors, has been developed.

It has been found that the connection site on medical devices comprising a neck element with guiding tracks having a locking edge to establish a good connection with a medical device is generally not good to use with second connection sites having threads or a engage/disengagement arrangement which operates by a turning motion. As both connection sites use a turning motion to connect or disconnect, such turning motion could accidentally disconnect a medical device to the medical device connector.

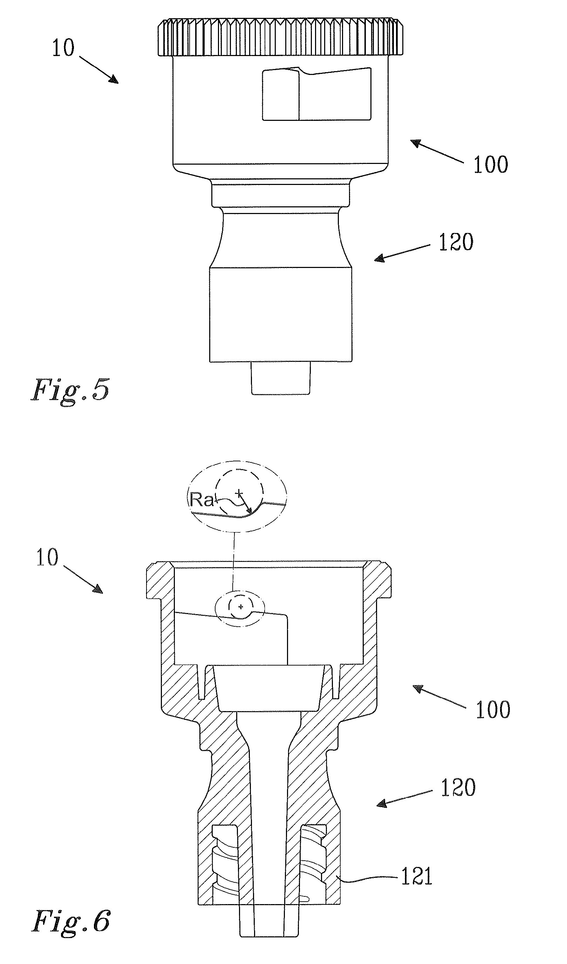

US5,139,483 and DE1005685 provide examples of medical connector devices. WO2008/136720 relates to a fluid transfer device for transferring fluid from a first fluid container to a second fluid container, such as a piercing member protection device. Fig. 5 shows a fluid transfer device 501 including a first connection part 502 comprising connection means 515 to connect the fluid transfer device 501 to a first fluid container, such as an injector. The locking edge includes a sharp inclined step in the upper edge of the guiding track. A flexible barrier member 548 is arranged in the first connection part 502.

### SUMMARY OF THE INVENTION

It is the objective of the present invention to remove or reduce the at least one of the above mentioned drawbacks. This is at least partly done by a medical device according to claim 1 comprising a first connection site for connecting a second medical device. The first connection site comprises at least one guiding track. The at least one guiding track is arranged with a surface comprising a locking edge. The locking edge is arranged to cooperate with a lock protrusion on the second medical device. The locking edge extends between a first and a second level. The locking edge further extends as a smooth curvature between the first and the second level, the curvature of which is a function of at least one radius. The present invention provides for easy usage of a medical device connector which can be smoothly connected or disconnected, locked or unlocked. The radius is preferably 1-10 mm, more preferably 2-8 mm or even more preferably 3-5 mm.

The medical device can be a medical device connector in which the medical device connector comprises a neck element, such as a cylinder like neck element, extending from a base member for receiving parts of said second medical device; the neck element comprises the at least one guiding track. Optionally the medical device can be a piercing member protection device. The guiding track 12 can in an embodiment be arranged on a sleeve member arranged in a telescopically manner with a second sleeve member.

In an embodiment according to the present invention, the distance between the first and the second level is between 0,2-3,0 mm, preferably between 0,2-1,0 mm.

The locking edge extends in a smooth curvature between the first and the second level. The curvature of which is a function of one radius, i.e. only one radius.

The guiding track of the neck element usually comprises a vertical section and a horizontal section arranged substantially perpendicular to the vertical section, and the horizontal section can comprise a distal and proximal surface, a first and a second vertical surface. It should be noted however that these two sections can in an embodiment be arranged with an angle of between 45-135° with respect to each other.

The radius has advantageously a centre of origin positioned at a distance from the second vertical surface of the guiding track. The distance is advantageously adapted to be between 3-20 mm. The smooth curvature is preferably initiated from a distance of 1-6 mm, preferably between 2-5 mm from the second vertical surface of the guiding track. In the shown embodiment the distance e is about 2,6 mm. This provides enough space for a lock protrusion of a mating medical device while at the same time keeping the lock protrusion snugly fitted in the guiding track.

The medical device connector can be arranged with at least two connection sites, e.g. it may comprise a second connection site for connecting to two medical devices. The second connection site can comprise threads, and in an embodiment be a male or female luer lock coupling.

In an embodiment according to the present invention, the medical device is a piercing member protection device comprising at least one guiding track. The piercing member protection device is preferably telescopically arranged, i.e. having a fist member and a second member being telescopically arranged with respect to each other. The telescopically function enables the piercing member to function between two positions in which the piercing member is either exposed or not exposed. The medical device comprises a barrier member arranged to cooperate with the lock protrusion of the second medical device so as to exert a force component to the second medical device.

### BRIEF DESCRIPTION OF THE DRAWINGS

The present invention will de described in greater detail with reference to the accompanying figures in which;
figure 1 shows a piercing device in the form of a piercing member protection device with a needle, a medical device connector and a vial; the medical device connector being connected to the vial;
figures 2a-2c show the medical device connector from figure 1 shown in different views;
figure 2d shows two medical device connectors, as shown in figure 1, piled in a stack of medical device connectors;
figure 3 shows a cross section of the medical device connector shown in figure 1;
figure 4a-4b shows parts of the guiding track of the medical device connector as shown in figure 1 in a more detailed view;
figures 5-6 show an alternative medical device having a first connection site, identical to the medical device connector shown in figure 1;
figure 7 shows an alternative medical device in the form of a piercing member protection device.

### DEFINITION

By the term "medical device" is meant a device used in hospital environments, nursing environments or care taking environments usually by qualified personnel such as doctors, nurses or the like. Such environments generally have high requirements regarding hygiene, personal care, and a strive towards low risk for contaminations. Typical medical devices are needles, syringes, piercing member protection devices, vials, infusion bags, infusion sets, administration systems, adapters, tubes, medical device connectors for connecting or adapting different medical devices to each other, or the like.

### DETAILED DESCRIPTION OF PREFERRED EMBODIMENTS

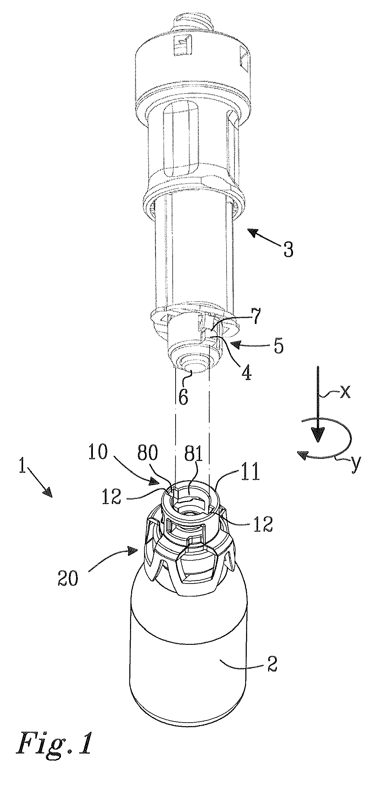

Figure 1 shows a medical device 1 in the form of a medical device connector 1 for connecting two medical devices. The medical devices can be a vial 2 and a piercing device 3. The piercing device 3 can be a piercing device having a telescopically movable piercing member protection function, as will be outlined below. The medical device connector 1 comprises a first connection site 10 adapted to receive and establish a connection with the piercing device 3 and a second connection site 20 adapted to establish a connection with the vial 2. The second connection site 20 operates by being fitted onto the neck of the vial 2 with a snap on function.

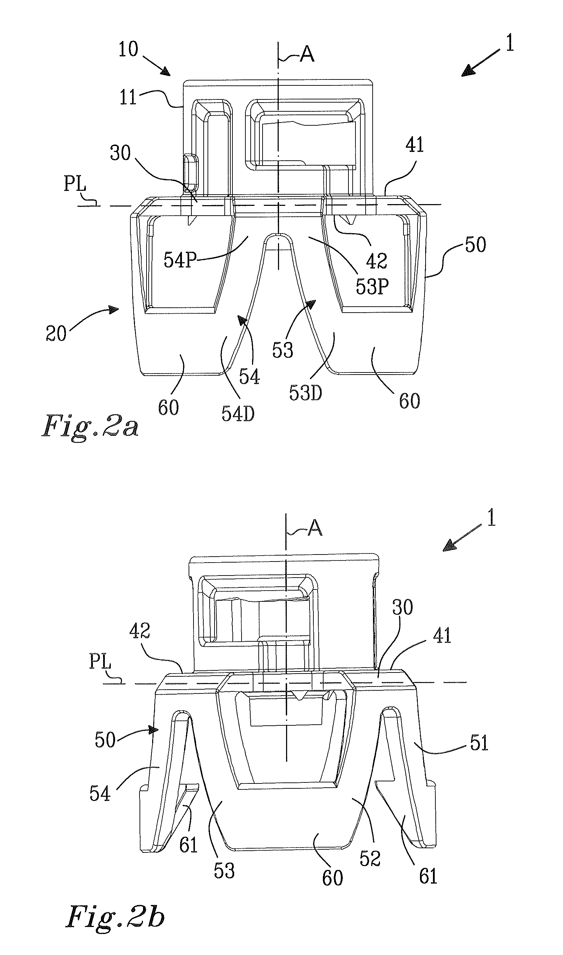

Figures 2a-2c show the medical device connector 1 in different views, the same feature is indicated with the same reference numeral. Figures 2a-2c show the first and the second connection site 10, 20 arranged on a base member 30. The medical device connector 1 has a centre axis A. The base member 30 separates the first and the second site 10, 20 from each other but is formed integrally with the first and the second connection site. The base member 30 has an extension in the plane PL, as indicated in figures 2a-2c.

A plurality of flanges 40 extends from the base member 30. The embodiment shown in figures 2a-2d has four symmetrically positioned flanges 40; a first, a second, a third and a forth flange 41, 42, 43, 44, extending parallel with the plane PL out from the periphery of the base member 30. The flanges 40 are formed integrally with the base member 30 but can be formed separately and connected thereto. A plurality of grip members 50 are arranged on the base member 30 via the flanges 40. In the shown embodiment, each flange member 41, 42, 43, 44 comprises two grip members 51, 52, 53, 54 (not all grip members are shown). The grip members 51, 52, 53, 54 are flexible and will deform somewhat as the they are connected to the vial 2, to thereafter return substantially to their original position after passing a flange on the vial 2, whereafter the grip members connect the medical device connector 1 to the vial 2 in a known "snap-on" manner.

Figure 2a shows a view towards the second flange 42 and the two grip members 53, 54 of the second flange 42. Each grip member 50 of the medical device connector 1 comprises a proximal end P and a distal end D, in figure 2a this is illustrated by the grip member 53 having a proximal end 53_{P} and a distal end 53_{D}. The proximal ends are nearer to the base member 30.

Between each adjacent grip member 52, 53 of separate flanges 41, 42, four bridge sections 60 are provided. As is noticed, the bridge sections extend from the distal ends of the grip members and thereby connect the distal ends 52_{D}, 53_{D} of the grip members 52, 53 of separate flanges 41, 42. Each bridge section 60 comprises a wedge portion 61 enabling a snap on function to the vial 1 shown in figure 1.

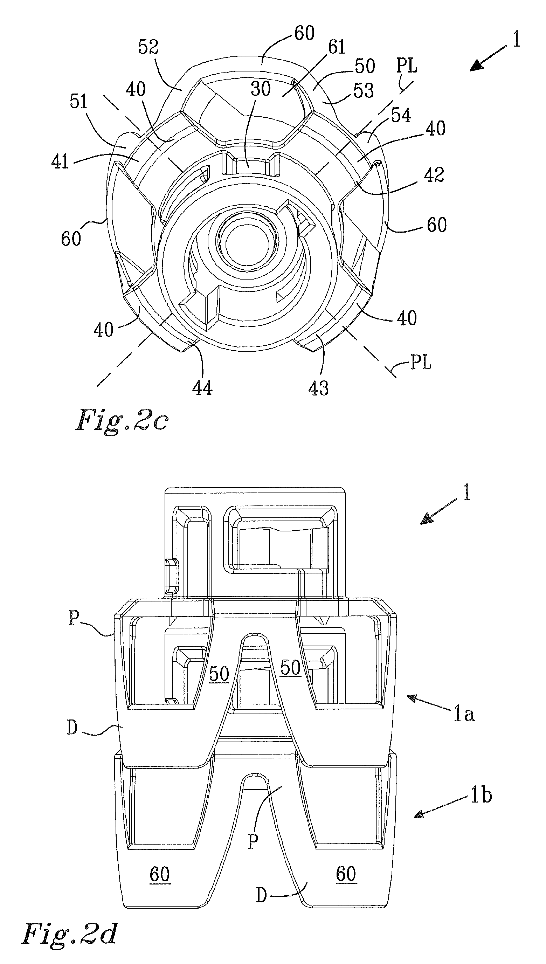

The distance between the proximal ends is smaller than the distance between the distal ends of the grip members. This provides for grip members having a somewhat tilted appearance and extending in a non parallel direction with respect to the centre axis A. This enables a plurality of medical device connectors 1 a, 1 b to be stacked in a relatively compact manner, as shown in figure 2d.

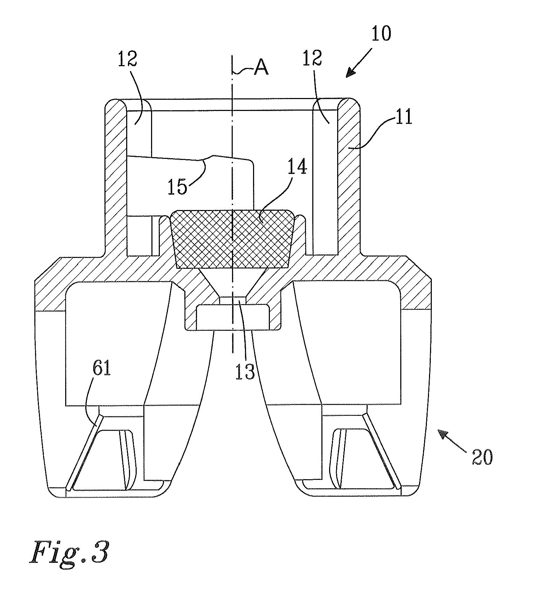

Figure 3 shows a cross section of the medical device connector 1, shown in figures 1, and 2a-2d. The first connection site 10 comprises a neck element 11 having two guiding tracks 12 (both shown in e.g. figure 2c) for receiving lock protrusions 4 of the piercing device 3, shown in figure 1. Each guiding track 12 comprises a locking edge 15. The lock protrusions 4 of the piercing device 3 cooperate with the locking edge 15 to connect the piercing device 3.

Intersecting with the centre axis A is a through going aperture 13 arranged to permit a needle of the piercing device 3 to extend therethrough after assembly and during use. A barrier member 14 made from e.g. silicone rubber material or a thermoplastic elastomer (TPE) is arranged to seal around such needle during use and to seal after use. The barrier member 14 covers the through going aperture.

Turning back to figure 1 again, the neck element 11 comprises two opposing guiding tracks 12, symmetrically positioned. The guiding tracks 12 exhibit an L-form, comprising a first vertical section 80 and a substantially horizontal section 81 arranged substantially perpendicular to the vertical section 80.

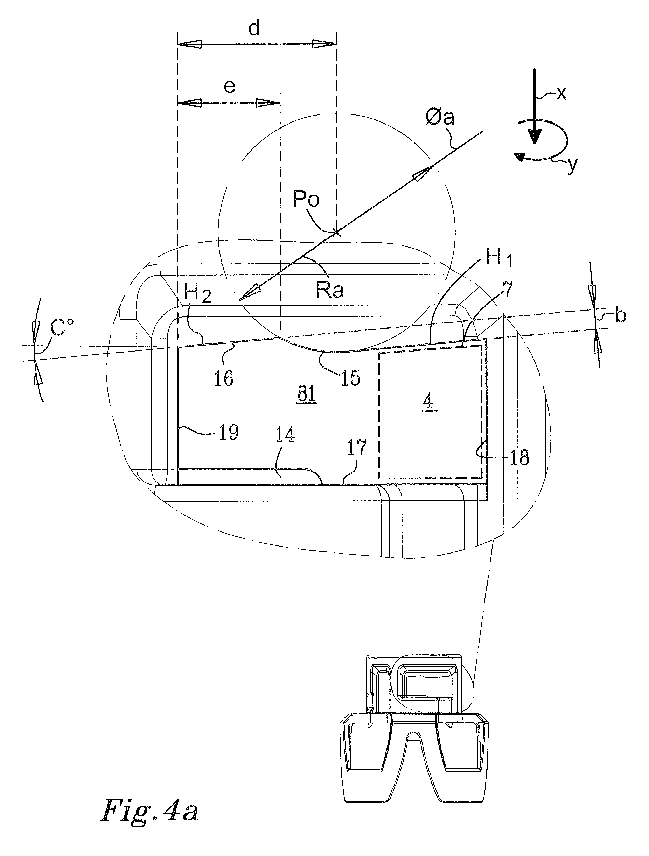

Figures 4a-4b show an enlargement of parts of the neck element 11 and one of the guiding tracks 12 of the first connection site 10 of the medical device connector 1 seen in figures 1-4. The guiding track 12 comprises the locking edge 15, or barrier section, which the lock protrusions 4 of the piercing device 3 are intended to cooperate with during assembly, as illustrated in figure 1. The tip 5 of the piercing device 3, with its barrier member 6 and lock protrusions 4, as shown in figure 1, is inserted into the neck element 11 of the first connection site 10. During the insertion, the lock protrusions 4 of the piercing device 3 slide in the vertical section 81 of the guiding track 12.

The arrows X, Y, shown in figures 1 and 4a-4b, show how the piercing device 3 is moved during insertion and locking, and in which order; X before Y. Disengagement is done in the opposite order and direction. First, with a vertical motion illustrated by arrow X, the tip 4 of the piercing device 3 is inserted so that the barrier member 6 of the piercing device 3 is positioned directly adjacent the barrier member 14 of the medical device connector 1, shown in figure 3. The barrier members 6, 14 are compressed by the vertical movement. When the lock protrusions 4 of the piercing device 3 are aligned with the vertical sections 81 of the guiding tracks 12, the piercing device 3 can be turned clockwise, as indicated by the arrow Y. During the clockwise turning, which in an embodiment of course can be counter clockwise should the guiding track 12 extend in that direction, the lock protrusion 4 is forced into the locked position. As is noticed, the neck element 11 comprises two guiding tracks 12 and the piercing device 3 comprises two lock protrusions 4, although each feature might be described in the singular. As the piercing member 3 is in the locked position, the needle can be exposed, penetrate the barrier members 6, 14, to provide for drug delivery or drug administration.

In figure 4a, parts of the lock protrusion 4 of the piercing device 3 are indicated with a dotted line and shown at the position before the turning motion, or the locking motion, i.e. before the motion indicated by arrow Y is performed. Figures 4a-4b also show the horizontal section 81 of the guiding track 12. As is noticed in figure 4a, the locking edge 15 smoothly extends in a smooth curvature between a first and a second level H1, H2, illustrated with the distance b in figure 4a-4b. The distance b can be 0,2-3 mm, preferably 0,2-1 mm. The locking edge 15 extends as a smooth curvature, the curvature of which is a function of a radius Ra, indicated by the diameter Øa. The radius Ra, being half of the diameter Øa. The radius Ra can be between 1-10 mm, preferably between 2-8 mm even more preferably between 3-5 mm. In the shown embodiment in figure 4a, the radius a is about 3 mm. The locking edge thus enables a good connection between the piercing device 3 and the medical device connector 1 which is easy to lock and unlock while still permitting a user to easily turn the piercing device 3 to a locked position, from the position indicated in figure 4a with the dotted lines of the lock protrusion 4. In an embodiment, the locking edge 15 can be extending in a smooth curvature, the curvature of which is the function of two radii, different or the same, but with different points of origin. The locking edge 15 thus extends smoothly between the two levels. By smoothly is meant a substantially continuous transition with no sharp edges.

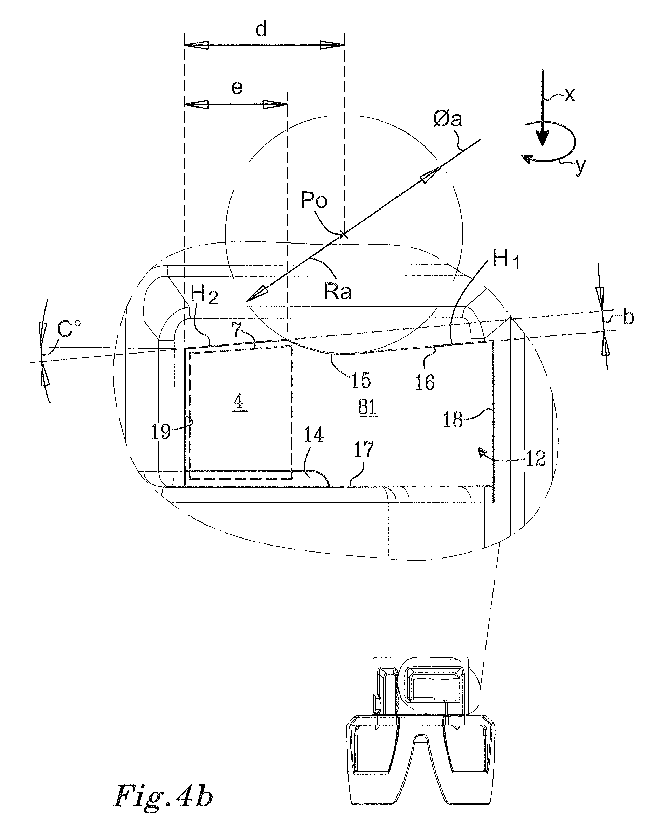

The horizontal section 81 of the guiding tracks 12 comprises a distal surface 16, a proximal surface 17, a first and a second vertical surface 18, 19, the distal surface 16 being further away from the base member 30, than the proximal surface 17. The locking edge 15 has a radius curvature, illustrated by arrow Ra in figures 4a and 4b. The radius Ra has a point of origin P_{O} at a distance d from the second side 19 of the horizontal section 81 of the guiding track 12, and starts at a distance e from the second side 19 of the horizontal section 81 of the guiding track 12. The distance d can be between 3-20 mm. The distance e can be between 1-6 mm, preferably between 2-5 mm. In the shown embodiment the distance e is about 2,6 mm. It should be noted that the distance d should be adapted after the radius Ra, distance e and the distance b.

The distal surface 16 of the guiding tracks 12 is further arranged with an angle c, as indicated in figure 4a-4b with respect to a proximal surface 17 of the guiding tracks 12. The proximal surface 17 of the guiding tracks 12 can be considered to be horizontal, or parallel with a still water line. The angle c is advantageously 0-15°, preferably 2-10°, even more preferably 5-7°. In the shown embodiment the angle c is 5°. Figures 4a-4b also show parts of the barrier member 14. The angled surface enables the piercing device 3 to be compressed towards the medical device connector 1 during assembly and the clockwise turning of the piercing device 3, as indicated by arrow Y in figure 4a as the lock protrusion 4 is moved towards the locking edge 15. The piercing device 3 is subjected to a counter force imparted by the compressed barrier member 14 and the compressed barrier member 6 of the piercing device 3 if such is present. The counter force exerts an upwardly directed force component on the piercing device 3 in a direction opposite to the arrow X.

As the lock protrusion 4 passes the locking edge 15, the upward force component forces a distal surface 7 of the lock protrusion 4 against the distal surface 16 of the guiding track 12 and thus keeps it in a locked position. Figure 4b shows the lock protrusion 4 in the locked position. Although referred to as the locked position, it is only locked form movement along the vertical arrow X.

The smooth curvature of the locking edge 15 enables a user to smoothly unlock, or more accurately, to smoothly pass the lock protrusion across the raised barrier which the locking edge 15 is composed of; thus enabling the unlocking of the piercing device from the first connection site of the medical device connector 1 to be performed simply, yet providing an effective locking function.

The present invention can be applied on a plurality of medical device connectors. Figures 5-6 show an alternative embodiment of a medical device connector 100 comprising a first and a second connection site 10, 120. The first connection site being the same first connection site 10 as described above. The second connection site 120 is a traditional male luer lock coupling 121. It should be noted that the second connection site could be a female luer lock coupling.

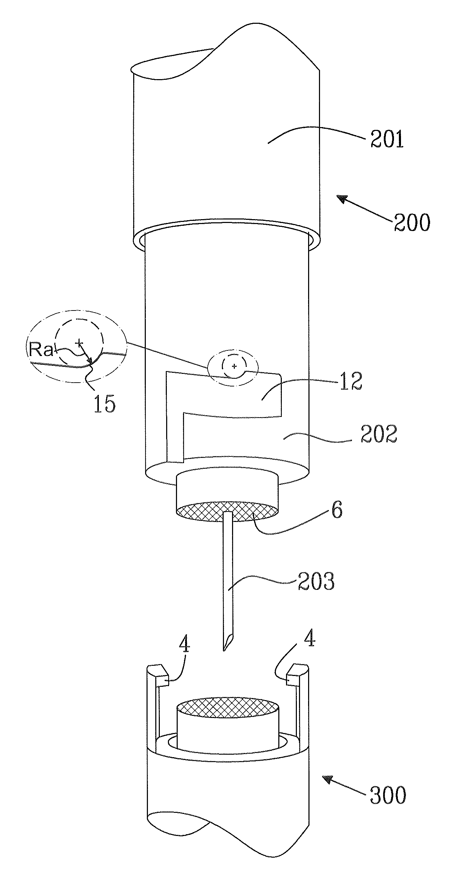

Figure 7 shows an alternative embodiment of the present invention. The guiding track 12, as described above, is in this embodiment arranged on a piercing member protection device 200. The piercing member protection device comprises a first and a second sleeve member 201, 202 and a piercing member 203. The first and a second sleeve members 201, 202 are telescopically arranged to each other such that when said first sleeve member 201 is in a first position, the piercing member 203 is not exposed, and when the first sleeve member 201 is in a second position, the piercing member 203 is exposed. In figure 7, the piercing member 203 is exposed and the first sleeve 201 is in its second position, with respect to the second sleeve 202. It should be noted that the second position, as seen in figure 7, with the piercing member 203 exposed is in practice only present after connection with the second medical device 300 and a full connection has been achieved.

The sleeve member 202 comprises at least one guiding track 12, preferably two guiding tracks 12. The guiding tracks 12 are arranged symmetrically on the side of eth sleeve 202 and are formed in the sleeve 202.

The barrier member 6 of the piercing member 200, and the barrier member 14 of the medical device connector 1, is arranged to cooperate with the said lock protrusions 4 of said second medical device 3, 300 so as to exert a force component to said second medical device. The force component helps to keep the lock protrusion in a locked position when the medical device has been connected to the second medical device.

## Claims

1. A medical device (1, 100, 200) having a vertical direction with an upper portion and a lower portion and a horizontal direction perpendicular to said vertical direction, said medical device comprising a first connection site (10) in said upper portion for connecting a second medical device (3) to said medical device, said first connection site (10) comprising;
at least one guiding track (12) for guiding a lock protrusion (4) on said second medical device (3),
said at least one guiding track (12) comprising at least a horizontal section (81), said horizontal section (81) having an upper portion and a lower portion in said vertical direction and comprising a distal surface (16) in said upper portion of said horizontal section (81) and a proximal surface (17) in said lower portion of said horizontal section (81), and a first and a second vertical surface (18, 19), said distal surface (16) comprising a locking edge (15), said locking edge (15) being arranged to cooperate with said lock protrusion (4) on said second medical device (3),
said medical device (1, 100, 200) comprising a barrier member (6,14) imparting a force component to said second medical device (3) to retain said lock protrusion (4) of said second medical device (3,300) towards said distal surface (16) and thus keep it in a locked position after assembly therewith,
**characterized in**
that said locking edge (15) extends in a smooth curve between a first and a second level (H1, H2) in said vertical direction, said curvature being a function of at least one radius (Ra).

2. The medical device (1, 100, 200) according to claim 1, **characterized in that** said radius (Ra) is between 1-10 mm.

3. The medical device (1, 100, 200) according to claim 1 or 2, **characterized in that** said distance (b) between said first and said second level (H1, H2) is 0,2-3,0 mm, preferably 0,2-1,0 mm.

4. The medical device (1, 100, 200) according to any preceding claims, **characterized in that** said locking edge (15) extends in a smooth curve between said first and said second level (H1, H2), said curvature is a function of one radius (Ra).

5. The medical device (1, 100, 200) according to claim 1, **characterized in that** said radius (Ra) has a centre of origin (P_{O}) positioned at a distance (d) from said second vertical surface (19) of said horizontal section (81) of said guiding track (12).

6. The medical device (1, 100, 200) according to any preceding claims, **characterized in that** said medical device comprises a base member (30) and a neck element (11) extending from said base member (30) for receiving a part of said second medical device (3) into said neck element (11), said neck element (11) comprising said at least one guiding track (12).

7. The medical device (1, 100, 200) according to claim 6, **characterized in that** said neck like element (11) is a cylinder like neck element (11).

8. The medical device (1, 100, 200) according to any one of the preceding claims, **characterized in that** said guiding track comprises a vertical section (80) arranged substantially perpendicular to said horizontal section (81), said vertical section arranged to initially receive said lock protrusion (4) during assembly.

9. The medical device (1, 100, 200) according to any preceding claims, **characterized in that** said medical device is a piercing member protection device (200) and **in that** said guiding track (12) is arranged on said piercing member protection device.

10. The medical device (1, 100, 200) according to 9, **characterized in that** said piercing member protection device (200) comprises a sleeve member (202) and **in that** said at least one guiding track (12) is formed in said sleeve member (202).

11. The medical device (1, 100, 200) according to any preceding claims, **characterized in that** said medical device (1, 100) comprises a second connection site (20, 120) for connecting an additional medical device.

## Patentansprüche

1. Vorrichtung für medizinische Zwecke (1, 100, 200) mit einer vertikalen Erstreckung mit einem oberen Abschnitt und einem unteren Abschnitt und mit einer horizontalen Streckung, die senkrecht zur vertikalen Erstreckung verläuft, wobei die Vorrichtung für medizinische Zwecke eine erste Verbindungsstelle (10) in dem oberen Abschnitt zum Anschluss einer zweiten Vorrichtung (3) für medizinische Zwecke an die Vorrichtung für medizinische Zwecke aufweist und wobei die erste Verbindungsstelle Folgendes aufweist:
mindestens eine Führungsbahn (12) zum Führen eines Verriegelungsvorsprungs (4) auf der zweiten Vorrichtung (3) für medizinische Zwecke,
wobei die mindestens eine Führungsbahn (12) mindestens einen horizontalen Bereich (81) aufweist, und wobei der horizontale Bereich (81) einen oberen Abschnitt und einen unteren Abschnitt in der vertikalen Erstreckung besitzt sowie eine distale Fläche (16) in dem oberen Abschnitt des horizontalen Bereichs (81) und eine proximale Fläche (17) in dem unteren Abschnitt des horizontalen Bereichs (81) und eine erste und eine zweite vertikale Fläche (18, 19), wobei die distale Fläche (16) eine Verriegelungskante (15) besitzt, wobei die Verriegelungskante (15) so angeordnet ist, dass sie mit dem Verriegelungsvorsprung (4) auf der zweiten Vorrichtung (3) für medizinische Zwecke zusammenwirkt,
wobei die Vorrichtung für medizinische Zwecke (1, 100, 200) ein Sperrenteil (6, 14) aufweist, das die zweite Vorrichtung (3) für medizinische Zwecke so mit einer Kraft beaufschlagt, dass der Verriegelungsvorsprung (4) der zweiten Vorrichtung (3, 300) für medizinische Zwecke zu der distalen Fläche (16) hin gesichert ist und damit nach Zusammenbau mit dieser in verriegelter Stellung gehalten wird,
**dadurch gekennzeichnet**,
dass die Verriegelungskante (15) sich zwischen einer ersten Höhe und einer zweiten Höhe (H1, H2) in der vertikalen Erstreckung entlang einer gleichmäßigen Kurve erstreckt, wobei die Krümmung eine Funktion mit mindestens einem Radius (Ra) ist.

2. Vorrichtung für medizinische Zwecke (1, 100, 200) nach Anspruch 1, **dadurch gekennzeichnet, dass** der Radius (Ra) zwischen 1 und 10 mm beträgt.

3. Vorrichtung für medizinische Zwecke (1, 100, 200) nach Anspruch 1 oder 2, **dadurch gekennzeichnet, dass** der Abstand (b) zwischen der ersten Höhe und der zweiten Höhe (H1, H2) 0,2 bis 3,0 mm, vorzugsweise zwischen 0,2 und 1,0 mm, beträgt.

4. Vorrichtung für medizinische Zwecke (1, 100, 200) nach einem der vorhergehenden Ansprüche, **dadurch gekennzeichnet, dass** sich die Verriegelungskante (15) entlang einer gleichmäßigen Kurve zwischen der ersten Höhe und der zweiten Höhe (H1, H2) erstreckt, wobei die Krümmung eine Funktion eines Radius (Ra) ist.

5. Vorrichtung für medizinische Zwecke (1, 100, 200) nach Anspruch 1, **dadurch gekennzeichnet, dass** der Radius (Ra) ein Ursprungszeitrum (P_{O}) aufweist, dass in einem Abstand (d) von der zweiten vertikalen Fläche (19) des horizontalen Bereichs (81) der Führungsbahn (12) positioniert ist.

6. Vorrichtung für medizinische Zwecke (1, 100, 200) nach einem der vorhergehenden Ansprüche, **dadurch gekennzeichnet, dass** die Vorrichtung für medizinische Zwecke ein Basisteil (30) und ein Halselement (11) aufweist, das sich von dem Basisteil (30) aus erstreckt und einen Teil der zweiten Vorrichtung (3) für medizinische Zwecke in dem Halselement (11) aufnimmt, wobei das Halselement (11) die mindestens eine Führungsbahn (21) umfasst.

7. Vorrichtung für medizinische Zwecke (1, 100, 200) nach Anspruch 6, **dadurch gekennzeichnet, dass** das halsartige Element (11) ein zylinderartiges Halselement (11) ist.

8. Vorrichtung für medizinische Zwecke (1, 100, 200) nach einem der vorhergehenden Ansprüche, **dadurch gekennzeichnet, dass** die Führungsbahn einen vertikalen Bereich (880) aufweist, der im Wesentlichen senkrecht zu dem horizontalen Bereich (81) angeordnet ist, wobei der vertikale Bereich so angeordnet ist, dass er während des Zusammenfügens den Verriegelungsvorsprung (4) anfänglich aufnimmt.

9. Vorrichtung für medizinische Zwecke (1, 100, 200) nach einem der vorhergehenden Ansprüche, **dadurch gekennzeichnet, dass** die Vorrichtung für medizinische Zwecke eine Vorrichtung (200) zum Schutz eines Einstechteils ist, und dass die Führungsbahn (12) auf der Vorrichtung zum Schutz eines Einstechteils angeordnet ist.

10. Vorrichtung für medizinische Zwecke (1, 100, 200) nach Anspruch 6, **dadurch gekennzeichnet, dass** die Vorrichtung zum Schutz eines Einstechteils (200) ein Hülsenteil (202) aufweist und dass die mindestens eine Führungsbahn (12) in dem Hülsenteil (202) ausgebildet ist.

11. Vorrichtung für medizinische Zwecke (1, 100, 200) nach einem der vorhergehenden Ansprüche, **dadurch gekennzeichnet, dass** die Vorrichtung für medizinische Zwecke (1, 100) eine zweite Verbindungsstelle (20, 120) für den Anschluss einer weiteren Vorrichtung für medizinische Zwecke aufweist.

## Revendications

1. Dispositif médical (1, 100, 200) présentant une direction verticale avec une partie supérieure et une partie inférieure et une direction horizontale perpendiculaire à ladite direction verticale, ledit dispositif médical comprenant un premier site de raccordement (10) sur ladite partie supérieure afin de raccorder un second dispositif médical (3) audit dispositif médical, ledit premier site de raccordement (10) comprenant :
au moins une piste de guidage (12) destinée à guider une saillie de verrouillage (4) sur ledit second dispositif médical (3),
ladite au moins une piste de guidage (12) comprenant au moins une section horizontale (81), ladite section horizontale (81) présentant une partie supérieure et une partie inférieure suivant ladite direction verticale et comprenant une surface distale (16) sur ladite partie supérieure de ladite section horizontale (81) et une surface proximale (17) sur ladite partie inférieure de ladite section horizontale (81), et des première et seconde surfaces verticales (18, 19), ladite surface distale (16) comprenant un bord de verrouillage (15), ledit bord de verrouillage (15) étant agencé de manière à coopérer avec ladite saillie de verrouillage (4) sur ledit second dispositif médical (3), ledit dispositif médical (1, 100, 200) comprenant un élément formant barrière (6, 14) appliquant une composante d'effort sur ledit second dispositif médical (3) afin de retenir ladite saillie de verrouillage (4) dudit second dispositif médical (3, 300) vers ladite surface distale (16) et de la maintenir ainsi dans une position verrouillée après assemblage avec ce dernier,
**caractérisé en ce que** ledit bord de verrouillage (15) s'étend suivant une courbe lisse entre un premier et un second niveau (H1, H2) dans ladite direction verticale, ladite courbure étant une fonction d'au moins un rayon (Ra).

2. Dispositif médical (1, 100, 200) selon la revendication 1, **caractérisé en ce que** ledit rayon (Ra) est compris entre 1 et 10 mm.

3. Dispositif médical (1, 100, 200) selon la revendication 1 ou 2, **caractérisé en ce que** ladite distance (b) entre ledit premier et ledit second niveaux (H1, H2) est comprise entre 0,2 et 3,0 mm, de préférence, entre 0,2 et 1,0 mm.

4. Dispositif médical (1, 100, 200) selon l'une quelconque des revendications précédentes, **caractérisé en ce que** ledit bord de verrouillage (15) s'étend suivant une courbe lisse entre ledit premier et ledit second niveaux (H1, H2), ladite courbure est une fonction d'un premier rayon (Ra).

5. Dispositif médical (1, 100, 200) selon la revendication 1, **caractérisé en ce que** ledit rayon (Ra) présente un centre d'origine (P0) positionné à une distance (d) par rapport à ladite seconde surface verticale (19) de ladite section horizontale (81) de ladite piste de guidage (12).

6. Dispositif médical (1, 100, 200) selon l'une quelconque des revendications précédentes, **caractérisé en ce que** ledit dispositif médical comprend un élément de base (30) et un élément formant col (11) s'étendant à partir dudit élément de base (30) afin de recevoir une partie dudit second dispositif médical (3) dans ledit élément formant col (11), ledit élément formant col (11) comprenant ladite au moins une piste de guidage (12).

7. Dispositif médical (1, 100, 200) selon la revendication 6, **caractérisé en ce que** ledit élément formant col (11) est un élément formant col cylindrique (11).

8. Dispositif médical (1, 100, 200) selon l'une quelconque des revendications précédentes, **caractérisé en ce que** ladite piste de guidage comprend une section verticale (80) agencée sensiblement perpendiculairement à ladite section horizontale (81), ladite section verticale étant agencée afin de recevoir initialement ladite saillie de verrouillage (4) au cours de l'assemblage.

9. Dispositif médical (1, 100, 200) selon l'une quelconque des revendications précédentes, **caractérisé en ce que** ledit dispositif médical est un dispositif de protection d'élément de perçage (200) et **en ce que** ladite piste de guidage (12) est agencée sur ledit dispositif de protection d'élément de perçage.

10. Dispositif médical (1, 100, 200) selon la revendication 9, **caractérisé en ce que** ledit dispositif de protection d'élément de perçage (200) comprend un élément formant manchon (202) et **en ce que** ladite au moins une piste de guidage (12) est formée sur ledit élément formant manchon (202).

11. Dispositif médical (1, 100, 200) selon l'une quelconque des revendications précédentes, **caractérisé en ce que** ledit dispositif médical (1, 100) comprend un second site de raccordement (20, 120) destiné à raccorder un dispositif médical supplémentaire.
